(19) Europäisches Patentamt
European Patent Office
Office européen des brevets

(11) Publication number: **0 127 387 A2**

(12) **EUROPEAN PATENT APPLICATION**

(21) Application number: 84303331.7

(22) Date of filing: 16.05.84

(51) Int. Cl.³: G 01 N 27/56
//G08B17/10

(30) Priority: 19.05.83 GB 8313846

(43) Date of publication of application: 05.12.84 Bulletin 84/49

(84) Designated Contracting States: DE FR GB

(71) Applicant: CITY TECHNOLOGY LIMITED
17/19 Sebastian Street
London EC1V 0HB(GB)

(72) Inventor: Tantram, Anthony Desmond Shand
50 Downsway
Great Bookham Surrey(GB)

(72) Inventor: Chan, Yat Shein
270 Norbury Avenue
London SW16(GB)

(74) Representative: Jennings, Guy Kenneth et al,
GILL JENNINGS & EVERY 53-64 Chancery Lane
London WC2A 1HN(GB)

# (54) Gas sensor.

(57) An electrolytic cell has a sensing electrode S, a counter electrode C and an auxiliary electrode A all in contact with the electrolyte of the cell. The electrodes S and C are connected in parallel, each in series with a respective variable resistor $R_S$, $R_A$, this parallel arrangement being connected in circuit with the electrode C to obtain a subtractive effect. The output signal $V_{net}$ from this circuit will be given by

$$V_{net} = iO_S R_S - iO_A R_A$$

where $iO_S$ and $iO_A$ are the base line currents flowing through the electrodes S and A respectively. If these currents are equal, then adjusting $R_S$ to be equal to $R_A$ will cancel the base line error completely. If the electrodes are not identical, adjustment of the relative values of $R_A$ and $R_S$ is necessary to obtain cancellation.

Fig. 1.

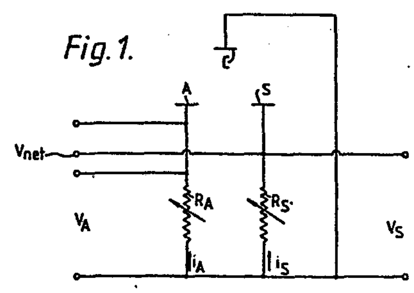


EP 0 127 387 A2

CITY TECHNOLOGY LIMITED



# GAS SENSOR

This invention relates to electro-chemical gas sensors in which the gas or vapour to be sensed is caused to react at an electrode of an electro-chemical cell to generate a current which is a function of the concentration of the gas or vapour to be sensed.

There is an increasing demand for gas sensors for safety monitoring, pollution control, fire detection, flue gas analysis, process control and so forth. In many cases, particularly for safety monitoring concerned with toxic gases where the safe limits are in the low ppm range, a high level of discrimination is required and it is important to reduce the effect of any interferences with the signal as far as possible. However, there are several factors which militate against such discrimination, one of which is the problem of a residual base line in the absence of reactant gas. This can be due to the presence of trace impurities or to side reactions of the catalysts used in the electrodes. The residual base line has a high temperature co-efficient, usually about doubling for every ten degrees centigrade rise in temperature. This means that although the base line may be electronically offset at one given temperature, it will inevitably manifest itself at different temperatures and so detract from the accuracy of the measurement, particularly

at low gas concentrations which are of importance in many applications such as the monitoring of toxic gases with low threshold limit values.

A further problem is that the gas sensor may not be completely specific. For example, the sensing electrode of the sensor may respond fully to more than one gas in a mixture.

According to the invention an electro-chemical cell has a first electrode, or normal sensing electrode which is exposed to the gas under test, a second, or auxiliary electrode, and a third, or counter electrode, all contacting the electrolyte of the cell, an electric circuit for effectively subtracting any desired fraction or multiple of the current passing through the auxiliary electrode from the current passing through the sensing electrode and means for effectively measuring the resulting net current to provide the basic signal from the sensor

If one is solely concerned with base line compensation the auxiliary electrode should have no gas access and should be chosen to be as similar as reasonably possible to the sensing electrode and such that the auxiliary electrode base line current is in the same direction as the sensing electrode base line current. In principle, the base line currents of the sensing and auxiliary electrodes will then be substantially equal and the result of the subtraction will be to virtually cancel out the base line. Provided the two electrodes are of the same material, their temperature behaviour will be similar and the cancellation will be maintained irrespective of temperature.

If the sensing electrode responds to two separate

gases in a mixture an auxiliary electrode with direct gas access should be chosen which reacts to the interfering gas with insignificant response to the gas of primary interest. The subtraction then results in a signal corresponding to the gas of primary interest. By separately monitoring the current from the auxiliary electrode a signal corresponding to the interfering gas may also be taken.

An alternative method of dealing with interfering gases is to provide for separate gas access to the auxiliary electrode via a filter which specifically removes the gas of primary interest. In this case the auxiliary electrode is chosen to be substantially identical to the sensing electrode. Subtraction of currents will result in a signal corresponding to the gas of primary interest. Again by separately monitoring the current from the auxiliary electrode a signal corresponding to the interfering gas or gases may also be taken.

If an interfering gas gives only a partial response at the sensing electrode, i.e. if the gas does not fully react so that a significant concentration remains at this electrode, a further approach may be used. In accordance with this approach, access of gas to the second or auxiliary electrode is provided via the sensing electrode, the gas diffusing over or preferably through the sensing electrode before

reaching the auxiliary electrode. The auxiliary electrode will not respond to any particular gas which fully reacts at the sensing electrode since it will be removed by the sensing electrode first. The auxiliary electrode will, however, respond to any other gas which reacts only partially at the sensing electrode. Such a partially reacting gas will, therefore, generate currents both at the auxiliary electrode and at the sensing electrode, and its effect will be cancelled out by subtraction of currents according to the invention. Again the current from the auxiliary electrode may be monitored separately to provide an additional signal corresponding to the partially interfering gas. The auxiliary electrode should be chosen to be as similar as reasonably possible to the sensing electrode so that its response to the partially interfering gas is similar. Exact matching may be difficult in practice, but the desired cancellation can be achieved by proportioning the two signals as required in the manner already described.

A gas sensor operating in this way is described in the co-pending application no: of even date herewith. In this form of sensor gas access to the auxiliary electrode via the primarily exposed electrode is preferably achieved by means of a partly hydrophobic, partly hydrophilic separator which permits the diffusion of gas while at the same time preserving the necessary electrolyte path between the electrodes in conjunction with known hydrophobic electrodes of the fuel cell type. The structure of such electrodes is that of a network of catalyst particle aggregates, which are hydrophilic and will wet up with electrolyte, interleaved with a network of porous PTFE, which is hydrophobic and will remain unwetted so providing gas paths throughout the depth of the electrode. Such electrodes are permeable to gas even when wetted with electrolyte.

Whatever the arrangement of electrodes, the sensing and auxiliary electrodes may, most simply, be connected in parallel with one another, each in series with a variable resistor. In practice it is very difficult to match electrodes precisely in order to obtain base line compensation, and the actual base lines are likely to be different. In this case the relative values of the variable resistors may be varied to achieve the necessary cancellation. It is also possible that the temperature co-efficients of the two base lines may not match exactly. In this case, the relative values of the resistors may be selected to give a zero or very low net base line temperature co-efficient and any net base line may be offset separately. The same result may be achieved with other types of circuitry. For example, the variable resistors may be replaced by current followers with adjustable gain. The output voltages from these may be adjusted relatively to one another as required before mixing to give the desired cancellation. Alternatively, the resistors may be fixed and be used with voltage followers of variable gain, the signals being mixed as desired after amplification.

An additional fourth or reference electrode may also be included in conjunction with a standard potentiostatic circuit. Any potential change occurring at the counter electrode due to polarisation will not then affect the potential of the sensing electrode.

The invention will now be described in more detail, with reference to the accompanying drawings, in which:-

Figures 1 and 2 are circuit diagrams of sensors operating in accordance with the invention;

Figure 3 is an exploded longitudinal sectional view of a compact sensor in accordance with the invention;

Figures 4A and 4B are a sectional view and bottom plan view respectively of a modified arrangement of electrodes for the sensor of Figure 3; and

Figures 5A and 5B are corresponding views of a component for use with the electrode arrangement of Figure 4.

In the circuit diagram of Figure 1, a cell is shown having a first or sensing electrode S, a second or counter electrode C and a third or auxiliary electrode A. The sensing and auxiliary electrodes are connected in parallel, each in series with a respective variable resistor $R_S$, $R_A$, this parallel arrangement being connected in circuit with the counter electrode C. If the base line currents flowing through the sensing and auxiliary electrodes respectively are denoted as $i^o{}_S$ and $i^o{}_A$ , it will be seen that the output signal $V_{net}$ from this circuit will be given by

$$V_{net} = i^o{}_S R_S - i^o{}_A R_A.$$

If the base line currents of the sensing and auxiliary electrodes are equal, then adjusting $R_S$ to be equal to $R_A$ will cancel the base line completely. Provided

that the auxiliary and sensing electrodes are effectively identical then their temperature behaviour will be the same and the cancellation will be maintained irrespective of temperature. On the other hand, it is difficult to match electrodes precisely and in practice the actual base lines may be different. In this case, the relative values of $R_A$ and $R_S$ need to be adjusted to achieve the necessary cancellation. it is also possible that the temperature co-efficients of the two base lines may not match exactly. In this case, the relative values of $R_A$ and $R_S$ need to be adjusted to give a zero or very low net base line temperature co-efficient, any net base line being offset separately.

The circuit of Figure 1 will also deal with problems arising due to side reactions such as minor surface oxidation or reduction of the electrode catalyst or reactions due to impurities in the electrolyte.

Thus in the circuit of Figure 1, the potential of the auxiliary electrode will follow that of the sensing electrode quite closely provided that the currents and resistance values are reasonably low and side reactions will be similar on the two electrodes. So any interfering currents of the type described above generated at the sensing electrode may be subtracted out exactly as described for the base line case.

The circuit of Figure 1 will also deal with cancellation of interfering gases by the methods described earlier. And, as already noted the separate signals $V_a$ and $V_S$, may be monitored across $R_a$ and $R_S$ respectively as well as the net signal $V_{net}$, as shown in Figure 1 Again the relative values of $R_A$ and $R_S$ may be adjusted to achieve the necessary cancellation. In some cases the $R_A$:$R_S$ ratio to cancel interference may differ from that to cancel base line and a compromise needs to be made dependent on the relative importance of these two criteria in the application concerned.

Figure 2 shows a similar circuit having all the characteristics already described with reference to Figure 1, but including an additional reference electrode R connected to a potential-controlling operational amplifier OA. If desired the circuit may also be used to apply a bias voltage $V_B$. The circuit will hold the potential between the reference electrode R and ground constant with the bias voltage $V_B$. It will be seen that the potential of the sensing electrode $V_S$ will now be given by

$$V_S = V_R + V_B - i_S R_S$$

so that the potential of the sensing electrode is now independent of any potential changes at the counter electrode. In principle $V_B$ can be made positive, zero or negative and the sign of $i_S$ will depend on whether the sensing electrode is acting as an anode or a cathode. The auxiliary electrode and its associated circuit is used exactly as described earlier to compensate for base line and any side effects from the residual potential change at the sensing electrode now equal to $i_S R_S$. It will also deal with the cancellation of interfering gases by the methods described earlier.

Figure 3 shows constructional details of a cell useful for operating in accordance with the circuit of Figure 1, although suitable for modification in accordance with the circuit of Figure 2.. This cell is designed to operate in accordance with the principle described earlier, namely by providing access for the gas mixture under test to the auxiliary electrode via the sensing electrode by means of the construction

of separator previously described. The three electrodes S, A and C are hydrophobic types electrodes in which the electrode catalyst is mixed with powdered PTFE and pressure-bonded to porous PTFE tapes 4, 5 and 6, the auxiliary electrode A and the counter electrode C having holes through their middles. The respective current connection leads to the electrodes are shown as 7, 8 and 9. The partly hydrophilic, partly hydrophobic porous insulating separator is shown as 10. Below the auxiliary electrode A is a conventional hydrophilic porous insulating separator 11 and a wick 12 of similar material which contacts the electrode S through the hole in the electrode A on PTFE tape 5 and passes through the holes in the separator 11, the counter electrode C on PTFE tape 6 and a hole 13 in a base plate 14 which provides an electrolyte reservoir 15.

The separator can conveniently be made, for example, by impregnating glass filter paper with a mixed suspension of silica powder and PTFE powder, drying and curing at 300 degrees centigrade. This will result in a separator with a network of hydrophilic channels through which electrolyte can penetrate, interleaved with a network of hydrophobic channels available for gas permeation. Other separator materials may, of course, be used together with other hydrophobic and hydrophilic materials, to achieve the same effect. An alternative method of making a partly hydrophobic, partly hydrophilic separator is to simply impregnate a glass filter paper separator in a number of restricted areas with PTFE suspension and dry and cure as before.

The impregnated areas will be hydrophobic and provide gas channels through the separator, and the unimpregnated areas are available to provide the electrolyte connection. Other forms of the separator are a hydrophilic separator with an annular ring of porous hydrophobic material or a hydrophilic separator with a hole, or holes filled with porous hydrophobic material. By mounting such a separator in close contact with the sensing electrode on one side and with the auxiliary electrode on the other, there will be a gas path right through the hydrophobic channels of the sensing electrode and the separator into the auxiliary electrode. The operating principles involved in the use of such a separator are described in more detail in the co-pending application referred to above.

If the cell is to be modified to operate in accordance with the circuit of Figure 2, the additional reference electrode may be included either above or below the counter electrode C. The other components necessary for completion of the cell include a compression O-ring 16 and a top plate 17 formed with a capillary diffusion barrier 18. The sensor is assembled by bolting up, using bolts through holes in the base plate 14 and top plate 17, two of which holes are shown at 19. After assembly, the reservoir 15 is partially filled with electrolyte and sealed off with a bottom cap (not shown). The electrolyte will then wick up into the various separators and electrodes. The separator 10 will be in close contact on the one side with the sensing electrode S and on the other side with the auxiliary electrode A so that there is a gas path

through the hydrophobic channels of the sensing electrode S and separator 10 into the auxiliary electrode A.

Accordingly, gas entering through the capillary 18 has direct access to the electrode S where it can at least partially react. Gas passing through the electrode S can then pass through the separator 10 and thus into contact with the electrode A, the reactions at the two electrodes being in accordance with the principles already described.

Instead of the capillary gas barrier 18, other types of diffusion barrier can be included if required, e.g. a porous membrane, a diffusion barrier having pores which are sufficiently small as to allow gas diffusion through them to be in accordance with the Knudsen principle or a non-porous membrane.

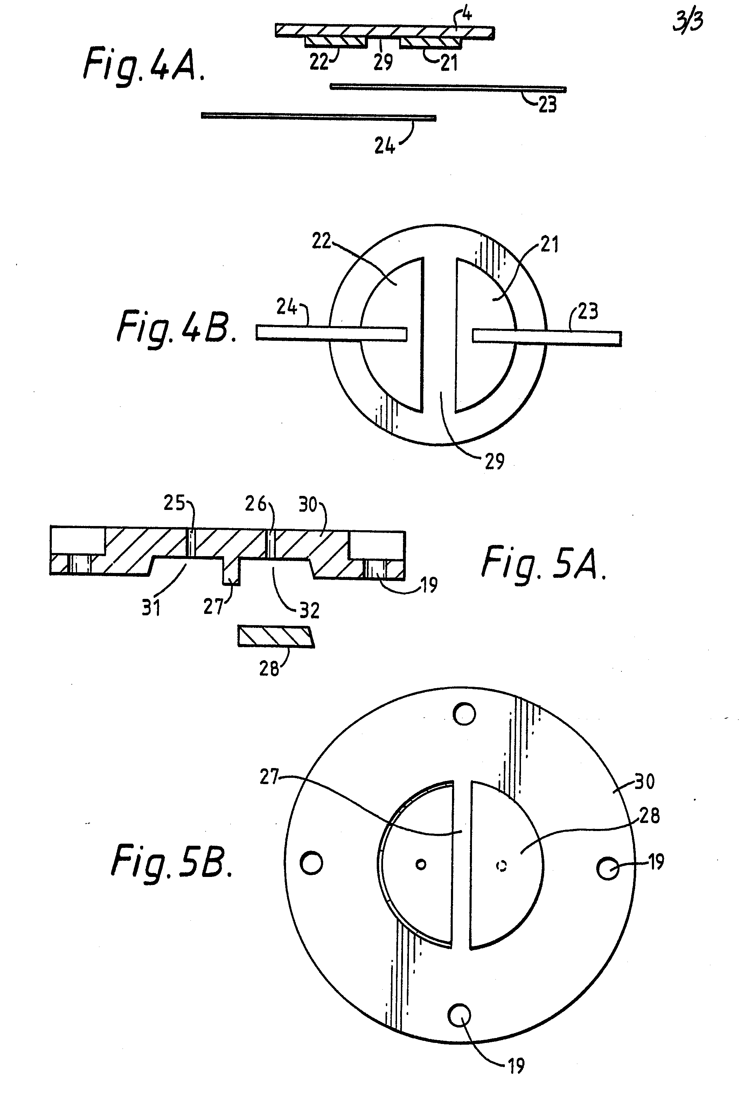

Figures 4A and 4B show a modified electrode arrangement for use when it is desired to provide gas access to the auxiliary electrode, as well as the sensing electrode, either directly or via a specific gas filter. In both these cases the separator 10 is replaced by a normal hydrophilic separator. Components corresponding to those of Figure 3 are indicated by the same reference numerals. Electrodes 21 and 22 which are approximately semi-circular in shape with a space 29 between them are mounted on the same porous PTFE tape 4 and have respective current connection leads 23 and 24. When both electrodes have direct gas access, they comprise different catalytic materials in order to yield different gas reactions, as already described. This double electrode component 21, 22 with its connection replaces the parts S, 4 and 7 in Figure 3.

The components A, 5, 8 forming the auxiliary electrode in Figure 3 may be omitted or alternatively may be included and used as a reference electrode with the circuit shown in Figure 2.

Figures 5A and 5B show a component 30 for use with the electrode arrangement of Figures 4A and 4B, where access to the auxiliary electrode is via a specific filter. The component 30 replaces the part 17 in Figure 3. The central capillary hole 18 of Figure 3 is replaced by two capillary holes 25 and 26 feeding respective compartments 31, 32 of a cavity which is divided into two by a cross piece 27. A filter element 28 shown exploded in Figure 5A fits into the compartment 32 of the divided cavity. As in Figure 3 bolting holes are shown as 19. On assembly the cross piece 27 fits into the space 29 shown in Figure 4 and is coated with a sealant that will penetrate porous PTFE to seal to the porous PTFE tape 4 to ensure a good seal between the divided compartments. Gas passing through the capillary 25 has independent direct access to the electrode 22 of Figure 4 while gas passing through the capillary 26 traverses the filter 28 before reaching the electrode 21.

The following are examples of sensors in accordance with the invention. In all these examples the electrodes are gas diffusion electrodes of the hydrophobic type which are pressure bonded to porous PTFE tape. Unless specifically stated otherwise the electrolyte is 10 normal sulphuric acid.

EXAMPLE 1

A sensor as illustrated in Figure 3, but with a conventional hydrophilic separator in place of the separator 10, was operated with the circuit of Figure 1. All the electrodes were of the hydrophobic type and based on platinum black. The relative weights of platinum in the three electrodes A, S and C were 1:2:4 respectively.

The capillary diffusion barrier 18 was 3 mm long and 2.0 mm diameter. When tested with carbon monoxide in air the signal $V_{net}$ was equivalent to a sensitivity of 0.11 µA per ppm.

A base line current measured between electrodes S and C, which gives the value typical of a conventional two-electrode cell was equivalent to 1.5 ppm carbon monoxide at 20 degrees centigrade and 6.5 ppm carbon monoxide at 40 degrees centigrade. When operated in accordance with the invention, with $V_{net}$ used as the signal, the base line at 20 degrees centigrade could be nulled out by setting the ratio of $R_A:R_S$ to 2.0. At this setting the base line at 40 degrees centigrade was only 1 ppm carbon monoxide equivalent.

EXAMPLE 2

A sensor designed to measure carbon monoxide with insignificant interference from hydrogen was constructed exactly as in Example 1, save only that it

had a partly hydrophobic partly hydrophilic separator 10. This had five hydrophobic channels each about 2.5 mm diameter made by impregnating a glass mat separator with PTFE suspension followed by drying and curing at 300 degrees centigrade.

When tested with hydrogen in air in the simulated conventional two-electrode mode, i.e. when the signal was taken across $R_S$ to measure the current between electrodes S and C, the hydrogen interference at 20 degrees centigrade was found to be 100 ppm of hydrogen equivalent to 40 ppm of carbon monoxide.

When operated in accordance with the invention with $V_{net}$ used as the signal it was found that the hydrogen interference could be reduced to zero by setting the ratio of $R_A:R_S$ to 1.9. Using $V_{net}$ the carbon monoxide sensitivity was equivalent to 0.12 µA per ppm.

Hydrogen could be monitored separately by taking a signal across $R_A$. Tested over the range 0 - 100 ppm the response was linear with a sensitivity of 0.025 µA per ppm.

## EXAMPLE 3.

A sensor was constructed in accordance with Example 2 except that the special separator 10 was made by totally impregnating a glass mat with an equal weight PTFE/silica powder suspension and drying and curing as before.

The sensor had broadly similar characteristics to that of Example 2, but the $R_A:R_S$ ratio required for eliminating hydrogen interference was found to be

1.6:1 and the sensitivity of the separate hydrogen signal monitored across $R_A$ was equivalent to 0.034 µA per ppm.

EXAMPLE 4.

A sensor as illustrated in Figure 3 but using the double electrode component illustrated in Figure 4 in place of electrode S was designed for the separate measurement of carbon monoxide and sulphur dioxide in air containing both of these gases. It was tested using the circuit of Figure 1. Electrode 22 of Figure 4, corresponding to S in Figure 1, had a platinum catalyst. Electrode 21 of Figure 4, corresponding to A in Figure 1, had a gold catalyst. While both carbon monoxide and sulphur dioxide will react on a platinum electrode, only sulphur dioxide will react on a gold electrode. The counter electrode was a platinum based air electrode. The electrolyte was fifty percent phosphoric acid.

The dimensions of the capillary 18 were 3 mm long and 2.1 mm diameter. It was found that when the ratio of $R_A$ to $R_S$ was set to 1.05 the net subtracted signal $V_{net}$ from the sensor was independent of the sulphur dioxide concentration giving a sensitivity to carbon monoxide equivalent to 0.1 µA per ppm.

In addition a separate signal was monitored across $R_A$ and found to be independent of carbon monoxide concentration, but linear with sulphur dioxide concentration with a sensitivity of 0.065 µA per ppm.

EXAMPLE 5

A gas sensor designed to measure both hydrogen and carbon monoxide was constructed according to Figure 3, but modified to include the double electrode component illustrated in Figure 4 instead of electrode S, together with the modified top illustrated in Figure 5 enabling a filter element 28 to be fitted. The sensor was further modified to include an air reference electrode R to enable it to be run with the circuit of Figure 2 which was used with zero bias.

Both the electrodes 21 and 22 of Figure 4 were platinum based, the filter element 28 of Figure 5 was fitted above electrode 21 which corresponded with electrode A in Figure 2. The adjacent electrode 22 corresponded with electrode S of Figure 2. The filter element was composed of equal weights of silver oxide and manganese dioxide intimately mixed with PTFE dispersion as a binder, dried and cured at 300 degrees centigrade. This material will catalytically remove carbon monoxide but not hydrogen from air, so that while electrode S sees both carbon monoxide and hydrogen, electrode A sees only hydrogen. The two separate capillary holes 25 and 26 were each 33 mm long and 2.5 mm diameter.

When the ratio of $R_A$ to $R_S$ was set to 1.2:1, it was found that the net subtracted signal $V_{net}$, was independent of hydrogen concentration and linear with carbon monoxide concentration showing a sensitivity equivalent to 0.11 µA per ppm. The signal across $R_A$ was monitored separately and found to be independent of

carbon monoxide concentration but dependent on hydrogen concentration with a sensitivity equivalent to 0.076 µA per ppm of hydrogen.

EXAMPLE 6

A sensor designed to monitor both sulphur dioxide and carbon monoxide in air was constructed and operated exactly as in Example 5 save only that the filter material used was manganese dioxide alone bonded with PTFE. This will remove sulphur dioxide from air but not carbon monoxide.

When the ratio of $R_A$ to $R_S$ was set to 1.15:1 it was found that the net subtractive signal was independent of carbon monoxide concentration with a sensitivity to sulphur dioxide equivalent to 0.07 µA per ppm of sulphur dioxide.

A separate signal monitored across $R_A$ was independent of sulphur dioxide concentration with a sensitivity to carbon monoxide equivalent to 0.12 µA per ppm of carbon monoxide.

## CLAIMS

1. A gas sensor comprising an electro-chemical cell having a first electrode, a second electrode and a counter-electrode all in contact with the electrolyte of the cell, of which at least the first electrode is accessible to a gas under test, whereby, in operation, respective currents flow between the counter-electrode and the first electrode and between the counter-electrode and the second electrode, and an electric circuit for effectively subtracting any desired fraction or multiple of the current passing through the second electrode from the current passing through the first electrode and means for effectively measuring the resulting net current to provide an output signal.

2. A gas sensor according to claim 1 in which the first electrode is directly accessible to the gas to be tested, while gas access to the second electrode is via a filter which specifically removes the gas of primary interest so that the effect of interfering gases is subtracted to give a net signal specific to the gas of primary interest.

3. A gas sensor according to claim 1 in which both the first and second electrodes are directly accessible to a gas under test, the second electrode having little response to the gas of primary interest which responds on the first electrode but both first and second electrodes respond to an interfering gas whereby the effect of the

interfering gas may be subtracted from the output signal to give a resultant signal substantially dependent only on the gas of primary interest.

4. A gas sensor according to claim 1 in which the arrangement of the first and second electrodes is such that gas access to the second electrode is via the first electrode.

5. A gas sensor according to claim 4 in which the gas passes through the first electrode before reaching the second electrode.

6. A gas sensor according to claim 5, including between the first electrode and the second electrode a porous insulating separator which is partly hydrophilic and partly hydrophobic.

7. A gas sensor according to claim 6, in which the separator comprises a network of hydrophilic channels through which electrolyte can penetrate interspersed with a network of hydrophobic channels for gas permeation.

8. A gas sensor according to claim 6, in which the separator comprises contiguous hydrophilic and hydrophobic areas.

9. A gas sensor according to any one of the preceding claims and also including a reference electrode connected to a potentiostatic circuit in order to compensate for potential changes due to polarisation.

10. A gas sensor according to any one of the preceding claims, in which the electric circuit also includes connection or connections for deriving signal or signals proportional to the separate first and/or second electrode currents.

11. A gas sensor according to any one of the preceding claims in which the first electrode and the second electrode are connected in parallel with one another each in series with a variable resistor for adjusting the relative values of the voltage signals generated by the two respective electrode currents.

12. A gas sensor according to any of claims 1 to 10 in which the potentials of the first electrode and/or the second electrode are independently controlled.

13. A sensor according to any one of the preceding claims additionally comprising a gas diffusion barrier in the path of the gas being sensed to the first electrode or to the second electrode or to both first and second electrodes.

14. A sensor according to claim 13, wherein the diffusion barrier is a capillary, a porous membrane, a diffusion barrier having pores which are sufficiently small as to allow gas diffusion through them to be in accordance with the Knudsen principle or a non-porous membrane.

Fig. 1.

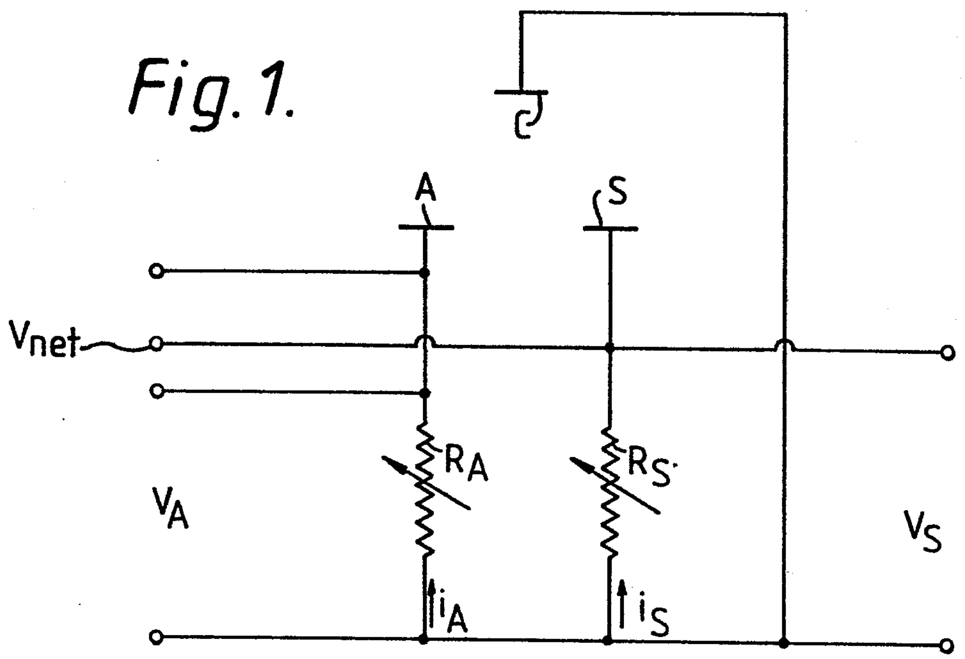


Fig. 2.

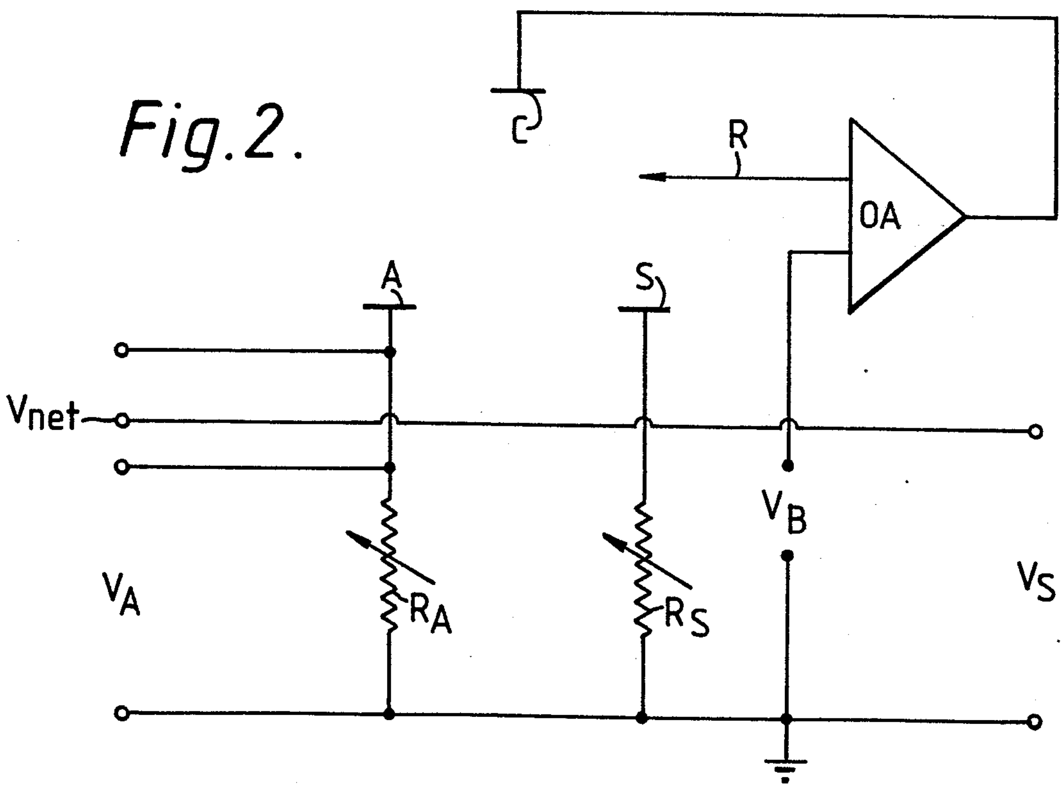

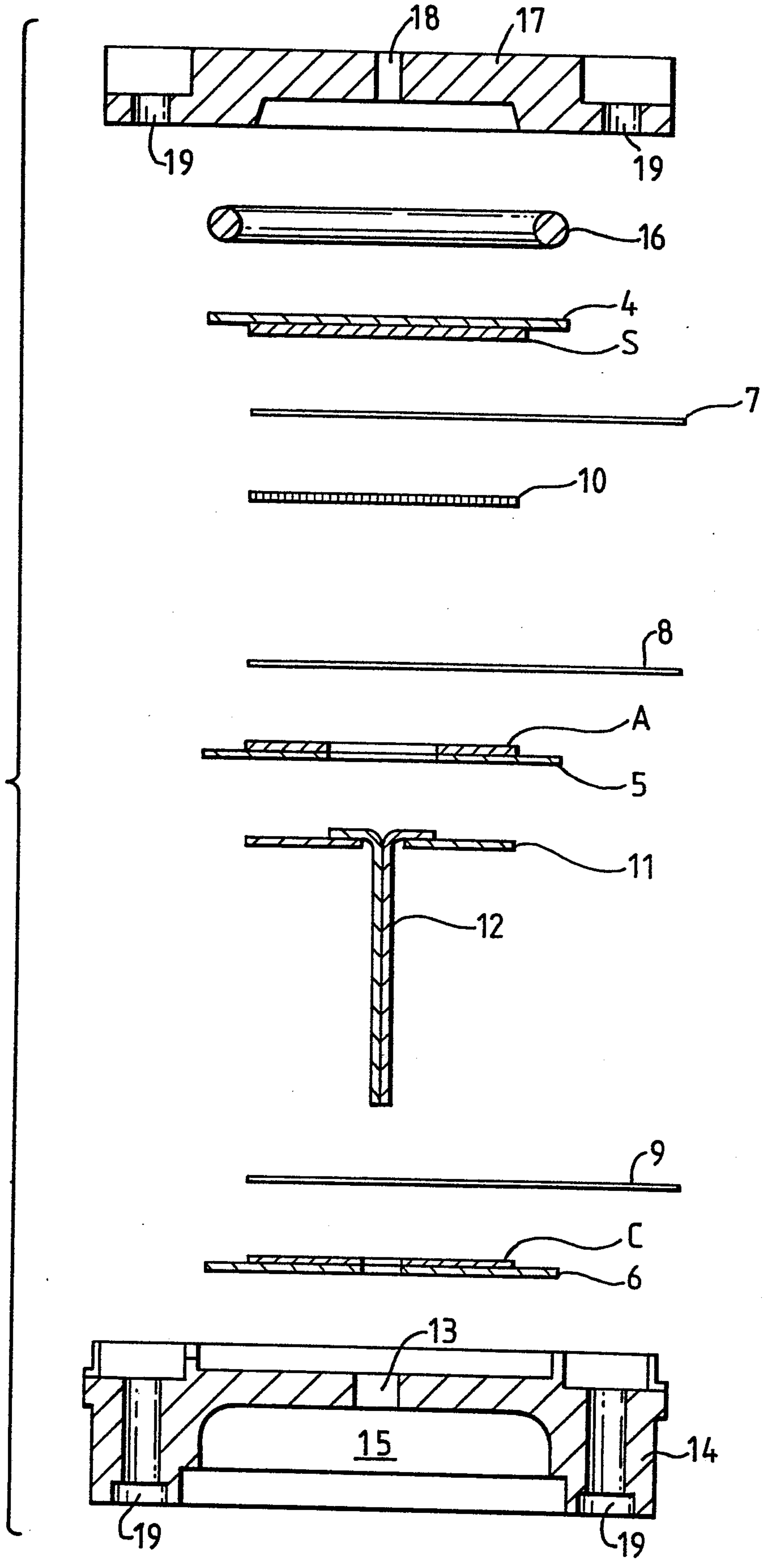


Fig. 3.

Fig. 4A.

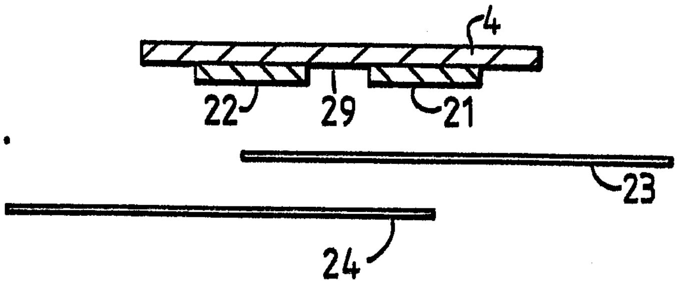


Fig. 4B.

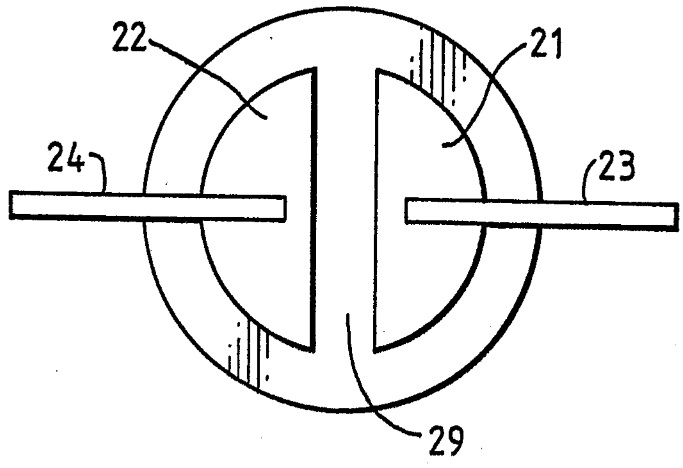


Fig. 5A.

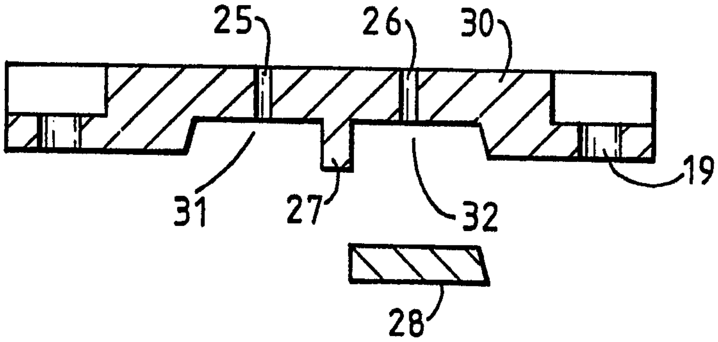


Fig. 5B.